Europäisches Patentamt

European Patent Office

Office européen des brevets

(11) Publication number: **0 307 914 B1**

# EUROPEAN PATENT SPECIFICATION

(45) Date of publication of patent specification: **29.07.92**　(51) Int. Cl.⁵: **A61K 31/70**

(21) Application number: **88115146.8**

(22) Date of filing: **15.09.88**

The file contains technical information submitted
after the application was filed and not included in
this specification

(54) **Pharmaceutical compositions containing a dideoxynucleoside.**

(30) Priority: **18.09.87 US 98255**

(43) Date of publication of application:
**22.03.89 Bulletin  89/12**

(45) Publication of the grant of the patent:
**29.07.92 Bulletin  92/31**

(84) Designated Contracting States:
**AT BE CH DE FR GB IT LI LU NL SE**

(56) References cited:
**EP-A- 0 206 497
EP-A- 0 216 510
EP-A- 0 216 511**

**J. MED. CHEM., vol. 30, May 1987, American
Chemical Society, Washington, DC (US); CH-
H.KIM et al., pp. 862-866**

**MOLECULAR PHARMACOLOGY, vol. 32, no. 1,
July 1987, The American Society for Phar-
macology and Experimental Therapeutics;
J.BALZARINIet al., pp. 162-167**

**J. AM. ACAD. DERMATOL., vol. 21; pp.
1213-1217**

(73) Proprietor: **F. HOFFMANN-LA ROCHE AG
Postfach 3255
CH-4002 Basel(CH)**

(72) Inventor: **Soo, Whaijen
169 Coolidge Terrace
Wyckoff, N.J. 07481(US)**

(74) Representative: **Lederer, Franz, Dr. et al
Patentanwalt Dr. Franz Lederer Lucile-
Grahn-Strasse 22
W-8000 München 80(DE)**

**Description**

The field of viral chemotherapeutics has recently developed in response to the particularly challenging problems presented with respect to the diagnosis and treatment of viral diseases. Of particular interest is the development of compounds effective against retroviruses, most particularly the HIV virus.

The effectiveness of any antiviral chemotherapeutic naturally depends on many factors including the identification of the specific virus, an understanding of its infectivity, life cycle, replication, and spread within the infected host.

Unfortunately, most of these antiviral substances which are nucleoside analogs are not specific inhibitors of only viral processes. Most of these compounds will interfere to a greater or lesser degree with normal molecular processes of the host cell resulting in toxic effects on uninfected cells.

This is particularly true with the 2',3' dideoxynucleoside analogs such as 2',3' dideoxycytidine (ddC) and the analogs thereof. ddC in particular causes peripheral neuropathy which results in tingling, numbing and pain which may require potent pain killers such as morphine when administered at the dosages which are effectively antiviral.

The instant invention comprises the discovery that extremely low dosages of dideoxynucleoside analogs exhibit no neuropathic effects yet are effective in preventing AIDS or treating subjects infected with the AIDS retrovirus.

The publication, J. Med. Chem. Vol. 30 p. 862-866 discloses that in in-vitro experiments ddC concentrations of 0.5 $\mu$M were noncytotoxic. In the document, EP-A-0206497 dosages of from 3,0-120 mg/kg/day of 2',3'-dideoxynucleosides are recommended in the treatment of viral and retroviral infections.

The instant invention relates to the use of 2',3'-dideoxycytidine in the manufacture of a medicament to provide a dosage unit containing 2',3-dideoxycytidine in an amount sufficient to administer to a subject from 0.001 to 0.05 mg/kg/day for the prevention or treatment of AIDS.

Particularly preferred is wherein 0.001 to 0.05 mg/kg 2',3'-dideoxycytidine is administered to a subject in one to six doses per day.

Most preferred is wherein 0.01 mg/kg 2',3'-dideoxycytidine is administered four times a day to a subject.

The compound may be administered orally, intravenously, parenterally, or mucocutaneously.

It is possible for the compounds of the present invention to be administered alone in solution. However, in the preferred embodiment, the active ingredient(s) may be used or administered in a pharmaceutical formulation. These formulations comprise at least one active ingredient (the dideoxynucleoside), together with one or more pharmaceutically acceptable carriers and/or other therapeutic agents. As included within the scope of this invention, "acceptable" is defined as being compatible with other ingredients of the formulation and not injurious to the patient or host cell. These carriers include those well known to practitioners in the art as suitable for oral, rectal, nasal, topical, buccal, sublingual, vaginal, or parenteral (including subcutaneous, intramuscular, intravenous, and intradermal) administration. Specific carriers suitable for use in the invention are further defined below.

In the present case, it will be appreciated that the compounds according to the invention may also be used in the manufacture of pharmaceuticals for the treatment or prophylaxis of viral infections.

The formulations may conveniently be presented in unit dosage form and may be prepared by and methods known in the pharmaceutical art. Such methods include the preparation of the active ingredient in a carrier which may contain additional medicinally active ingredients.

One method of oral administration of the 2',3'-dideoxycytidines of the present invention consists of dissolving an effective amount of the 2',3'-dideoxycytidine in a sodium chloride solution, preferably 0.9% sodium chloride in orange juice. The preferred method is administration of the 2',3'-dideoxynucleosides in tablet form, and may include one or more of the following: lactose (hydrous, fast flow), microcrystalline cellulose, colloidal silicon dioxide, croscarmellose sodium, magnesium stearate, stearic acid, and other excipients, colorants, and pharmacologically compatible carriers. Compositions for oral use may be administered to patients in fasting or non-fasting state. Examples of tables formulations are indicated in Tables 1 and 2.

Formulations of the present invention suitable for oral administration (including sustained release formulations) may be presented as discrete units such as capsules, cachets or tablets, each containing a predetermined amount of the active ingredient; as a powder or granules; as a solution or a suspension in an aqueous liquid; in an oil-in-water liquid emulsion or a water-in-oil liquid emulsion. The active ingredient may also be presented.

Formulations suitable for topical administration include lozenges comprising the active ingredient in a flavor, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising, for example, cocoa butter or a salicylate.

Formulations suitable for vaginal administration may be presented as pessaries, tampons, creams, gels, pastes, foams, or spray formulas containing in addition to the active ingredient such carriers as are known in the art to be appropriate.

Formulations suitable for parenteral administration include aqueous and non-aqueous, isotonic sterile injection solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampules and vials, and may be stored in a freeze-dried (lyophilized) condition requiring only the addition of the sterile liquid carrier, for example, water for injections, immediately prior to use. Extemporaneous injection solutions and suspensions may be prepared from sterile powders, granules and tablets of the kind previously described.

The administered ingredients may also be used in therapy in conjunction with other anti-viral drugs and biologicals, or in conjunction with other immune modulating therapy including bone marrow or lymphocyte transplants or medications.

A particularly preferred composition contains in one dosage unit 0.01 to 5 mg of 2',3'-dideoxycytidine.

Pharmaceutical compositions may be prepared by conventional formulation technique using the ingredients outlined below:

| 1. 0.01 mg Tablet | |
| --- | --- |
| Ingredients | mg/tablet |
| 1. 2',3'-dideoxycytidine | 0.01 |
| 2. Lactose, Anhydrous | 93.99 |
| 3. Iron Oxide Yellow | 0.10 |
| 4. FD&C Blue #1 Lake | 0.10 |
| 5. Microcrystalline Cellulose (Avicel® PH-102) | 25.00 |
| 6. Croscarmellose Sodium, Type A (Ac-Di-Sol®) | 5.00 |
| 7. Magnesium Stearate | 0.80 |
| Total | 125.00 mg |

| 2. 0.05 mg Tablet | |
| --- | --- |
| Ingredients | mg/tablet |
| 1. 2',3'-dideoxycytidine | 0.05 |
| 2. Lactose, Anhydrous | 94.00 |
| 3. Iron Oxide Black | 0.15 |
| 4. Microcrystalline Cellulose (Avicel® PH-102) | 25.00 |
| 5. Croscarmellose Sodium, Type A (Ac-Di-Sol®) | 5.00 |
| 6. Magnesium Stearate | 0.80 |
| Total | 125.00 mg |

| 3. 0.1 mg Tablet | |
|---|---|
| Ingredients | mg/tablet |
| 1. 2',3'-dideoxycytidine | 0.10 |
| 2. Lactose, Anhydrous | 149.25 |
| 3. Red Oxide #7067 | 0.15 |
| 4. Microcrystalline Cellulose (Avicel® PH-102) | 40.00 |
| 5. Croscarmellose Sodium, Type A (Ac-Di-Sol®) | 8.40 |
| 6. Magnesium Stearate | 2.10 |
| Total | 200.00 mg |

| 4. 0.5 mg Tablet | |
|---|---|
| Ingredients | mg/tablet |
| 1. 2',3'-dideoxycytidine | 0.50 |
| 2. Lactose, Anhydrous | 149.00 |
| 3. Microcrystalline Cellulose (Avicel® PH-102) | 40.00 |
| 4. Croscarmellose Sodium, Type A (Ac-Di-Sol®) | 8.40 |
| 5. Magnesium Stearate | 2.10 |
| Total | 200.00 mg |

| 5. 1.5 mg Tablet | |
|---|---|
| Ingredients | mg/tablet |
| 1. 2',3'-dideoxycytidine | 1.50 |
| 2. Lactose, Anhydrous | 148.50 |
| 3. Microcrystalline Cellulose (Avicel® PH-102) | 40.00 |
| 4. Croscarmellose Sodium, Type A (Ac-Di-Sol®) | 8.40 |
| 5. Magnesium Stearate | 1.60 |
| Total | 200.00 mg |

| 6. 2 mg Tablet | |
|---|---|
| Ingredients | mg/tablet |
| 1. 2',3'-dideoxycytidine | 2.00 |
| 2. Lactose, Anhydrous | 147.00 |
| 3. Cosmetic Iron Oxide Yellow | 0.40 |
| 4. Microcrystalline Cellulose (Avicel® PH-102) | 40.00 |
| 5. Croscarmellose Sodium, Type A (Ac-Di-Sol®) | 8.40 |
| 6. Magnesium Stearate | 2.00 |
| Total | 200.00 mg |

| 7. 5 mg Tablet | |
| --- | --- |
| Ingredients | mg/tablet |
| 1. 2',3'-dideoxycytidine | 5.00 |
| 2. Lactose, Anhydrous | 144.20 |
| 3. FD&C Blue #1 Lake | 0.10 |
| 4. Microcrystalline Cellulose (Avicel® PH-102) | 40.00 |
| 5. Croscarmellose Sodium, Type A (Ac-Di-Sol®) | 8.40 |
| 6. Magnesium Stearate | 2.30 |
| Total | 200.00 mg |

B. Sterile Powder for Injection

| 1. Lyophilized Powder, 10 mg/Vial | |
| --- | --- |
| Ingredients | mg/vial |
| 1. 2',3'-dideoxycytidine | 10.00 |
| 2. Mannitol | 50.00 |
| 3. Hydrochloric Acid (1% v/v) | |
| 4. Water for Injection | |
| Note: 1. The Hydrochloric Acid solution was used to adjust the pH of the bulk solution prior to lyophilization. 2. The Water for Injection is essentially volatilized during lyophilization. | |

C. Powder for Reconstitution

## 1. <u>Powder for Reconstitution, 0.02 mg/ml and 0.5 mg/ml when reconstituted</u>

Concentration when Reconstituted:     0.02 mg/ml  0.5 mg/ml

<u>Ingredients</u>

| | | 0.02 mg/ml | 0.5 mg/ml |
|---|---|---|---|
| 1. | 2',3'-dideoxycytidine | 0.002 | 0.050 |
| 2. | Potassium Sorbar | 0.200 | 0.200 |
| 3. | D&D Red #33 | 0.006 | 0.006 |
| 4. | FMC Corp. Raspberry Flavor #05695 | 0.030 | 0.030 |
| 5. | Citric Acid | 0.050 | 0.050 |
| 6. | Sodium Citrate | 0.180 | 0.180 |
| 7. | Lactose, Anhydrous | 5.000 | 5.000 |
| 8. | Surcrose | <u>30.000</u> | <u>30.000</u> |
| | Total | 35.468 g | 35.516 g |

<u>To Reconstitute</u>:

| | 0.02 mg/ml | 0.5 mg/ml |
|---|---|---|
| Distilled Water to be added | 76 ml | 76 ml |
| Final volume obtained | 100 ml | 100 ml |
| Reconstituted concentration | 0.02 mg/ml | 0.5 mg/ml |

D. <u>Syrup</u>

| Ingredients | 0.02 mg/ml | 0.5 mg/ml |
|---|---|---|
| | g/100 ml | |
| 1. 2',3'-dideoxycytidine | 0.002 | 0.05 |
| 2. Sorbitol | 35.00 | 35.00 |
| 3. Glycerin | 10.00 | 10.00 |
| 4. Methylparaben | 0.18 | 0.18 |
| 5. Propylparaben | 0.02 | 0.02 |
| 6. D&C Red #33 | 0.005 | 0.005 |
| 7. FMC Corp. Raspberry Flavor #11070 | 0.30 ml | 0.30 ml |
| 8. Sodium Phosphate Dibasic | 0.30 | 0.30 |
| 9. Purified Water | qs 100.00 ml | qs 100.00 ml |
| Final pH | 7.5 | 7.5 |

**Claims**

1. The use of 2',3'-dideoxycytidine in the manufacture of a medicament to provide a dosage unit

6

containing 2',3-dideoxycytidine in an amount sufficient to administer to a subject from 0.001 to 0.05 mg/kg/day for the prevention of treatment of AIDS.

**Revendications**

1.  Application de 2' ,3'-didésoxycytidine à la préparation d'un médicament pour fournir une unité posologique contenant de la 2',3'-didésoxycytidine en une quantité suffisante aux fins d'administration à un sujet à raison de 0,001 à 0,05 mg/kg/jour pour la prévention ou le traitement du SIDA.

**Patentansprüche**

1.  Die Verwendung von 2',3'-Dideoxycytidin bei der Herstellung eines Medikaments zur Bereitstellung einer Dosierungseinheit mit einer für die Verabreichung von 0.001 bis 0,05 mg/kg/Tag an einen Patienten zwecks Verhütung oder Behandlung von AIDS hinreichenden Menge 2',3'-Dideoxycytidin.